# EUROPÄISCHE PATENTSCHRIFT

(11) **EP 3 229 752 B1**
(45) Veröffentlichungstag und Bekanntmachung des Hinweises auf die Patenterteilung: **21.10.2020**
(21) Anmeldenummer: 15790096.0
(22) Anmeldetag: 02.11.2015
(51) Int. Cl.: A61K 8/25, A61K 8/34, A61K 8/41, A61Q 5/02, A61Q 5/06, A61K 8/73, A61K 8/04

(54) **KOSMETISCHES PRODUKT ENTHALTEND ETHANOL UND EINE STÄRKEVERBINDUNG IN EINER VORRICHTUNG ZUR ENTSPANNUNGSVERDAMPFUNG**
COSMETIC PRODUCT CONTAINING ETHANOL AND A STARCH COMPOUND IN A DEVICE FOR FLASH EVAPORATION
PRODUIT COSMÉTIQUE CONTENANT DE L'ÉTHANOL ET UN COMPOSÉ D'AMIDON DANS UN DISPOSITIF DE VAPORISATION ÉCLAIR

(30) Priorität: 10.12.2014 DE 102014225421
(43) Veröffentlichungstag der Anmeldung: 18.10.2017
(73) Patentinhaber: Henkel AG & Co. KGaA, 40589 Düsseldorf (DE)
(72) Erfinder: KNAPPE, Thorsten, 22869 Schenefeld (DE); BAYERSDÖRFER, Rolf, 22589 Hamburg (DE); FÖRSTER, Thomas, 40591 Düsseldorf (DE)
(86) Internationale Anmeldenummer: PCT/EP2015/075374
(87) Internationale Veröffentlichungsnummer: WO 2016/091461

(56) Entgegenhaltungen:
- EP-A1- 1 932 503
- JP-A- 2007 319 234
- US-A- 3 372 840
- US-A1- 2002 074 349
- US-A1- 2002 079 377
- US-A1- 2004 065 683
- US-A1- 2013 018 333
- US-A1- 2013 330 282
- US-A1- 2014 000 643
- DATABASE GNPD [Online] MINTEL; 30. Mai 2010 (2010-05-30), Anonymous: "Dry Shampoo", XP002752421, gefunden im www.gnpd.com Database accession no. 1335274
- DATABASE GNPD [Online] MINTEL; 31. März 2005 (2005-03-31), anonymous: "Clear spry N (Unscented)", XP002752422, gefunden im www.gnpd.com Database accession no. 349568
- DATABASE GNPD [Online] MINTEL; 2. Januar 2015 (2015-01-02), anonymous: "Back to life dry shampoo", XP002752423, gefunden im www.gnpd.com Database accession no. 2880331

## Beschreibung

Die Anmeldung betrifft das technische Fachgebiet der Behandlung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur Haarbehandlung Gegenstand der vorliegenden Anmeldung

Eine ansprechend aussehende Frisur wird heute allgemein als unverzichtbarer Teil eines gepflegten Äußeren angesehen. Ein wesentlicher Aspekt der Haarpflege ist die Haarreinigung.

Im Bereich der Reinigung und Pflege keratinhaltiger Fasern hat die Sprühapplikation entsprechender kosmetischer Zubereitungen eine gewisse Bedeutung, wobei die Zubereitungen in der Regel als Pumpsprays oder Aerosolsprays appliziert werden. Hierzu werden die kosmetischen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel. Aufgabe der vorliegenden Erfindung war es daher, spezifische haarkosmetische Zubereitungen zur Reinigung und Pflege keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens eine gute Reinigungs- und Pflegewirkung zu realisieren. Es hat sich gezeigt, dass zur Lösung dieser Aufgabe aus der Vielzahl bekannten haarkosmetisch wirksamen Zubereitungen insbesondere alkoholische Stärkezubereitungen geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 40 bis 80 Gew.-% Ethanol;
   a2) 20 bis 60 Gew.-% mindestens einer Stärkeverbindung
b) eine Vorrichtung zur Entspannungsverdampfung der kosmetischen Zubereitung a) mit
   b1) einem Behälter, welcher einen geschlossenen Innenraum definiert, in dem die kosmetische Zubereitung aufgenommen werden kann,
   b2) einem Ventil oder einem vergleichbar wirkenden Schließelement, um den wenigstens anteilsweise mit der kosmetischen Zubereitung a) befüllten Innenraum des Behälters zu verschließen und zu öffnen,
   b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
   b4) einer Düse, welche eine Zerstäubung der aus dem Behälter entweichenden kosmetischen Zubereitung a) ermöglicht,
c) einen Vorratsbehälter für die kosmetische Zubereitung a), aus welchem die kosmetische Zubereitung a) in den Behälter b1) gelangen kann, wobei
   - der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
   - der Vorratsbehälter mindestens das Zehnfache Volumen, vorzugsweise mindestens das Fünfzigfache Volumen des Behälters b1) aufweist,
   - der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Die kosmetische Zubereitung a) ist vorzugsweise flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Die erfindungsgemäße kosmetische Zubereitung enthält als ersten wesentlichen Bestandteil 40 bis 80 Gew.-% Ethanol. Entsprechende Mittel zeichnen sich durch eine gute kosmetische Wirkung bei gleichzeitig guter Applizierbarkeit aus.

Ein zweiter wesentlicher Bestandteil erfindungsgemäßer kosmetischer Zusammensetzungen ist die Stärkeverbindung a2). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil der Stärkeverbindung am Gesamtgewicht der kosmetischen Zubereitung a) 10 bis 70 Gew.-%, vorzugsweise 20 bis 60 Gew.-% beträgt. Überraschenderweise lassen sich auch die erfindungsgemäß hohen Gewichtsanteile der Stärkeverbindung mittels der Vorrichtung zur Entspannungsverdampfung problemlos versprühen. Dies gelingt insbesondere auch ohne dass eine eventuell eingesetzte Düse verstopft.

Der zweite wesentliche Bestandteil erfindungsgemäßer kosmetischer Zubereitungen a) ist die Stärkeverbindung a1). Unter der Bezeichnung "Stärke" wird dabei ist ein Reservekohlenhydrat verstanden, welche von vielen Pflanzen in Form von üblicherweise 1 bis 200 µm großen Stärkekörnern (Granula) in verschiedenen Pflanzenteilen, z.B. in Knollen oder Wurzeln, Getreidesamen, Früchten sowie im Mark gespeichert wird.

Vorzugsweise liegt die Stärkeverbindung a2) als in dem Ethanol a1) dispergierte Feststoffpartikel vor. Unter "Feststoffpartikel" werden bei 20°C und 1013,25 mbar partikulär vorliegende Feststoffe verstanden.

Eine erfindungsgemäß bevorzugt verwendbare Stärkeverbindung wird ausgewählt unter mindestens einem - gegebenenfalls modifizierten - Polykondensationsprodukt von D-Glucose erhalten aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok. Besonders bevorzugt enthält das erfindungsgemäße Mittel mindestens eine Stärkeverbindung, die Tapioka Stärke, Kartoffelstärke, Maisstärke oder Reisstärke ist oder sich davon ableitet. Gemische der vorgenannten Stärkeverbindungen sind erfindungsgemäß ebenso umfasst.

Ganz besonders bevorzugt ist die Stärkeverbindung Reisstärke. Stärkeverbindungen auf Grundlage von Reisstärke sind beispielsweise unter den Bezeichnung Remy DR KA (INCI Bezeichnung: Oryza Sativa (Rice) Starch, CAS Nummer 9005-25-8) von der Firma Bene O Remy Industries oder unter der Bezeichnung Rice Starch D.S.A. 7 (INCI Bezeichnung: Oryza Sativa (Rice) Starch, Cetrimonium Chloride; CAS Nummer 9005-25-8) von der Firma Agrana erhältlich.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck po, zum Beispiel den umgebenden Luftdruck (po = 1,000 bar), entlastet werden kann, wodurch mindestens teilweise eine Verdampfung der kosmetischen Zubereitung a) erreicht wird.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Neben den zuvor beschriebenen Bestandteilen a1) und a2) kann die kosmetische Zubereitung a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Ein erstes Beispiel für einen bevorzugten Wirk- und Hilfsstoff sind die kationischen Tenside a3). Bevorzugte kationische Tenside a3) sind ausgewählt unter quartären Ammoniumverbindungen, Esterquats und Amidoaminen. Die kationischen Tenside sind in der kosmetischen Zubereitung a), bezogen auf deren Gesamtgewicht, in Mengen von 0,01 bis 4,0 Gew.-%, vorzugsweise 0,05 bis 2,0 Gew.-%, und insbesondere 0,1 bis 1,0 Gew.-% enthalten. Kationsiches Tenside a3) aus der Gruppe der quartären Ammoniumverbindungen sind besonders bevorzugt.

Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Ganz besonders bevorzugte erfindungsgemäße kosmetische Zubereitungen a) sind dadurch gekennzeichnet, dass sie bezogen auf ihr Gesamtgewicht 0,01 bis 4,0 Gew.-%, vorzugsweise 0,05 bis 2,0 Gew.-%, und insbesondere 0,1 bis 1,0 Gew.-% (C₁₂ bis C₁₈)-Alkyltrimethylammoniumsalz(e) enthalten.

Eine zweite Gruppe optionaler Wirk- und Hilfsstoffe bilden die hydrophob modifizierten Metalloxidpulver a4). Im Hinblick auf die kosmetische Wirkung hat es dabei als vorteilhaft erwiesen, wenn der Gewichtsanteil des hydrophob modifizierten Metalloxidpulvers am Gesamtgewicht der kosmetischen Zubereitung a) 0,01 bis 3,0 Gew.-%, vorzugsweise 0,05 bis 2,5 Gew.-% und insbesondere 0,1 bis 2,0 Gew.-% beträgt.

Unter "hydrophob modifiziert" oder "hydrophobiert" sind im Sinne der Erfindung solche Metalloxide zu verstehen, die zumindest an der Oberfläche der Partikel derart modifiziert wurden, dass das modifizierte Teilchen weniger von Wasser benetzt wird als das nicht modifizierte Teilchen. Insbesondere sind silanisierte, hydrophobierte Metalloxide bevorzugt. Als Reagenz zur Silanisierung des Metalloxids eignet sich erfindungsgemäß bevorzugt mindestens ein Vertreter der Gruppe, die gebildet wird, aus Silanen, Halogensilanen, Alkoxysilanen und Silazanen. Bevorzugt geeignete hydrophobierte Metalloxide des hydrophobierten Metalloxidpulvers werden erfindungsgemäß ausgewählt aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid. Für die Herstellung der erfindungsgemäßen kosmetischen Mittel besonders geeignet haben sich hydrophobierte Silikate erwiesen, wobei durch Silanierung oder durch Umsetzung mit Polydimethylsiloxan nachbehandelte pyrogene Kieselsäure besondere Vorteile aufweisen.

Die erfindungsgemäßen kosmetischen Zubereitungen a) enthalten vorzugsweise hydrophobiertes Siliciumdioxid. Die Art des hydrophob modifizierten Siliciumdioxids a4) ist nicht prinzipiell beschränkt, jedoch wird aufgrund der kosmetischen Eigenschaften entsprechender Mittel als hydrophobiertes Metalloxidpulver vorzugsweise mindestens ein silanisiertes, hydrophobiertes Siliziumdioxid eingesetzt.
Als Reagenz zur Silanisierung des Siliziumdioxids eignen sich erfindungsgemäß bevorzugt mindestens ein Vertreter der Gruppe, die gebildet wird, aus Silanen, Halogensilanen, Alkoxysilanen und Silazanen.
Bevorzugte Vertreter der Gruppe der Silane sind Hexa(C₁-C₂₀)alkyldisilane, insbesondere Hexamethyldisilan.
Wenn ein Halogensilan als Silylierungsmittel Anwendung findet, wird als bevorzugtes Halogensilan mindestens eine Verbindung aus der Gruppe ausgewählt, die gebildet wird, aus den Verbindungen

[(C₁-C₂₀)Alkyl]_{z'}SiX_{(4-z')}

X₃Si[(CH₂)ₙ-R]

X₂[(C₁-C₂₀)Alkyl]Si(CH₂)ₙ-R

[(C₁-C₂₀)Alkyl]_{(y'1)}[R-(CH₂)ₙ]_{(2-y')}SiX

worin
X ein Chlor-, Brom- oder lodatom bedeutet,
z' eine Zahl 1, 2 oder 3 ist,
y' eine Zahl 0, 1 oder 2 ist
n eine ganze Zahl von 1 bis 20 ist und
R steht für einen Rest aus
   (C₁-C₁₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 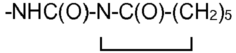 -NH-C(O)O-Me, -NH-C(O)O-Et, -NH-(CH₂)₃-Si(O(C₁-C₆)alkyl)₃.
Wenn ein Alkoxysilan als Silylierungsmittel verwendet wird, wird als bevorzugtes Alkoxysilan mindestens eine Verbindung aus der Gruppe ausgewählt, die gebildet wird, aus den Verbindungen

[(C₁-C₂₀)AlkylO]_{z}Si(C₁-C₂₀)Alkyl_{(4-z)}

[(C₁-C₂₀)AlkylO]_{z}Si[(CH₂)ₙ-R]_{(4-z)}

[(C₁-C₂₀)AlkylO]₂[(C₁-C₂₀)Alkyl]Si(CH₂)ₙ-R

[(C₁-C₂₀)AlkylO][(C₁-C₂₀)Alkyl]₂Si(CH₂)ₙ-R

[(C₁-C₂₀)AlkylO][(C₁-C₂₀)Alkyl]Si[(CH₂)ₙ-R]₂

(C₁-C₂₀Alkyl)₃SiO-C(CH₃)=N-Si(C₁-C₂₀)Alkyl₃,

worin
n eine ganze Zahl von 1 bis 20 ist und
z eine Zahl 1, 2, oder 3 bedeutet
R steht für einen Rest aus
   (C₁-C₂₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 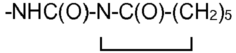 -NH-C(O)O-Me, -NH-C(O)O-Et, -NH-(CH₂)₃-Si(O(C₁-C₆)alkyl)₃
Als bevorzugtes Silazan wird mindestens eine Verbindung aus der Klasse der Disilazane, insbesondere mindestens eine Verbindung aus Disilazanen der Formel

R'₂R"Si-NH-SiR'₂R"

ausgewählt, worin
R' eine (C₁-C₂₀)Alkylgruppe bedeutet und
R" eine (C₁-C₂₀)Alkylgruppe oder eine Vinylgruppe bedeutet. Ein besonders bevorzugtes Silazan ist Hexamethyldisilazan.
Alle oben genannten Alkylgruppen, ob (C₁-C₆)Alkyl, (C₁-C₁₀)Alkyl oder (C₁-C₂₀)-Alkyl, können sowohl zyklisch, als auch linear bzw. verzweigt sein. Beispiele für erfindungsgemäß verwendbare Alkylgruppen sind Methyl, Ethyl, n-Propyl, Isopropyl, n-Butyl, Cyclopentyl, Cyclohexyl, n-Decyl, Lauryl, Myristyl, Cetyl, Stearyl, Isostearyl und Behenyl.
Ein Beispiel für eine erfindungsgemäße Arylgruppe ist die Phenylgruppe.
Beispiele für eine erfindungsgemäße (C₁-C₆)Perfluoroalkylgruppe sind Trifluormethyl, Perfluoroethyl, Perfluoropropyl und Perfluorohexyl.
Vorzugsweise werden hydrophobierte Siliziumdioxide eingesetzt, die durch Silanisierung von pyrogenem Siliziumdioxid erhalten werden.
Silanisierte, hydrophobierte Siliziumdioxide werden insbesondere bevorzugt ausgewählt aus mindestens einer Verbindung der Gruppe, die gebildet wird aus Trimethylsilylat-beschichtetem Siliziumdioxid, Dimethylsilylat-beschichtetem Siliziumdioxid, Octylsilylat-beschichtetem Siliziumdioxid.

Bevorzugte kosmetische Zubereitungen sind dadurch gekennzeichnet, dass das hydrophob modifizierte Metalloxidpulver a4) aus der Gruppe der durch Silanisierung von pyrogenem Siliciumdioxid erhaltenen hydrophobierten Silikate ausgewählt ist.

Eine Vielzahl geeigneter hydrophob modifizierter Siliciumdioxide ist kommerziell erhältlich. Beispielhaft seien Aerosil® R104 V, Aerosil® R106, Aerosil® R202, Aerosil® R805, Aerosil® R812, Aerosil® R812S, Aerosil® R972 und Aerosil® R8200, alle Degussa, sowie HDK® H2000, HDK® H2050 und HDK® H3004, alle Wacker, genannt. Ganz besonders bevorzugt kommt das Siliciumdioxid mit der INCI-Bezeichnung Silica Dimethyl Silylate zum Einsatz, das von der Firma Degussa unter der Bezeichnung Aerosil® R972 vertrieben wird. Zusammenfassend werden kosmetische Zubereitungen a) bevorzugt, bei denen das hydrophob modifizierte Metalloxidpulver a1) ausgewählt ist aus den Verbindungen mit den INCI Bezeichnungen Silica Dimethyl Silylate (z.B. Aerosil R792, Aerosil R794), Silica Dimethicone Silylate (z.B. Aerosil R202) und Silica Silylate (z.B. Aerosil R805, Aerosil R812, Aerosil R816). Besonders bevorzugt werden Verbindugen mit der INCI Bezeichnung Silica Dimethyl Silylate.

Die hydrophob modifizierten Siliciumdioxide mit der INCI Bezeichnung Silica Dimethyl Silylate ermöglich eine, im Vergleich zu den übrigen zuvor beschriebenen hydrophob modifizierten Siliciumdioxiden, vereinfachte Herstellung erfindungsgemäßer kosmetischer Mittel, welche sich zudem durch eine verbesserte Applizierbarkeit und kosmetische Wirkung auszeichnen. Die Herstellung der besonders bevorzugten hydrophobierten Siliciumdioxide mit der INCI Bezeichnung Silica Dimethyl Silylate kann beispielsweise durch Umsetzung pyrogener Kieselsäure mit Dimethyldichlorsilan erfolgen. Ein besonders bevorzugter Gegenstand der vorliegenden Anmeldung ist daher ein kosmetisches Mittel, welches ein hydrophob modifiziertes Metalloxidpulver enthält, das durch Umsetzung pyrogener Kieselsäure mit Dimethyldichlorsilan erhalten wird.

Der Partikeldurchmesser der Primärteilchen bevorzugter hydrophob modifizierter Metalloxide a4), insbesondere der hydrophob modifizierten Metalloxide mit der INCI Bezeichnung Silica Dimethyl Silylate beträgt vorzugsweise weniger als 5 µm, besonders bevorzugt weniger als 1 µm, und insbesondere zwischen 1 und 50 nm.

Bevorzugt sind weiterhin solche hydrophob modifizierten Metalloxide a4), insbesondere hydrophob modifizierte Metalloxide mit der INCI Bezeichnung Silica Dimethyl Silylate, die eine spezifische Oberfläche nach BET zwischen 10 und 400 m²/g, vorzugsweise zwischen 40 bis 300 m²/g und insbesondere 80 bis 150 m²/g aufweisen.

Als weitere geeignete Wirk- oder Hilfsstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| Ethanol a1) | 20 bis 95 | 30 bis 90 | 40 bis 80 | 73 | 33 |
| Stärkeverbindung a2) | 5 bis 80 | 10 bis 70 | 20 bis 60 | 26 | 66 |
| optionale Additive | add 100 | add 100 | add 100 | add 100 | add 100 |

| | Formel 6 | Formel 7 | Formel 8 | Formel 9 | Formel 10 |
|---|---|---|---|---|---|
| Ethanol a1) | 20 bis 95 | 30 bis 90 | 40 bis 80 | 73 | 33 |
| Oryza Sativa (Rice) Starch | 5 bis 80 | 10 bis 70 | 20 bis 60 | 26 | 66 |
| optionale Additive | add 100 | add 100 | add 100 | add 100 | add 100 |

| | Formel 11 | Formel 12 | Formel 13 | Formel 14 | Formel 15 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| kationisches Tensid a3) | 0,01 bis 4,0 | 0,05 bis 2,0 | 0,1 bis 1,0 | -- | 0,03 |
| optionale Additive | add 100 | add 100 | add 100 | add 100 | add 100 |

| | Formel 16 | Formel 17 | Formel 18 | Formel 19 | Formel 20 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| kationisches Tensid a3) | 0,01 bis 4,0 | 0,05 bis 2,0 | 0,1 bis 1,0 | -- | 0,03 |
| optionale Additive | add 100 | add 100 | add 100 | add 100 | add 100 |

| | Formel 21 | Formel 22 | Formel 23 | Formel 24 | Formel 25 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| (C₁₂ bis C₁₈)-Alkyltrimethylammoniumsalze | 0,01 bis 4,0 | 0,05 bis 2,0 | 0,1 bis 1,0 | -- | 0,03 |
| optionale Additive | add 100 | add 100 | add 100 | add 100 | add 100 |

| | Formel 26 | Formel 27 | Formel 28 | Formel 29 | Formel 30 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| (C₁₂ bis C₁₈)-Alkyltrimethylammoniumsalze | 0,01 bis 4,0 | 0,05 bis 2,0 | 0,1 bis 1,0 | -- | 0,03 |
| optionale Additive | add 100 | add 100 | add 100 | add 100 | add 100 |

| | Formel 31 | Formel 32 | Formel 33 | Formel 34 | Formel 35 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| Cetyltrimethylammoniumsalze | 0,01 bis 4,0 | 0,05 bis 2,0 | 0,1 bis 1,0 | -- | 0,03 |
| optionale Additive | add 100 | add 100 | add 100 | add 100 | add 100 |

| | Formel 36 | Formel 37 | Formel 38 | Formel 39 | Formel 40 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| Cetyltrimethylammoniumsalze | 0,01 bis 4,0 | 0,05 bis 2,0 | 0,1 bis 1,0 | -- | 0,03 |
| optionale Additive | add 100 | add 100 | add 100 | add 100 | add 100 |

| | Formel 41 | Formel 42 | Formel 43 | Formel 44 | Formel 45 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| hydrophobiertes Metalloxidpulver a4) | 0,01 bis 3,0 | 0,05 bis 2,5 | 0,1 bis 2,0 | 1,4 | -- |
| optionale Additive | add 100 | add 100 | add 100 | add 100 | add 100 |

| | Formel 46 | Formel 47 | Formel 48 | Formel 49 | Formel 50 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| hydrophobiertes Metalloxidpulver a4) | 0,01 bis 3,0 | 0,05 bis 2,5 | 0,1 bis 2,0 | 1,4 | -- |
| optionale Additive | add 100 | add 100 | add 100 | add 100 | add 100 |

| | Formel 51 | Formel 52 | Formel 53 | Formel 54 | Formel 55 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| silanisiertes pyrogenes Siliciumdioxid a4) | 0,01 bis 3,0 | 0,05 bis 2,5 | 0,1 bis 2,0 | 1,4 | - |
| optionale Additive | add 100 | add 100 | add 100 | add 100 | add 100 |

| | Formel 56 | Formel 57 | Formel 58 | Formel 59 | Formel 60 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| silanisiertes pyrogenes Siliciumdioxid a4) | 0,01 bis 3,0 | 0,05 bis 2,5 | 0,1 bis 2,0 | 1,4 | - |
| optionale Additive | add 100 | add 100 | add 100 | add 100 | add 100 |

| | Formel 61 | Formel 62 | Formel 63 | Formel 64 | Formel 65 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| Silica Dimethyl Silylate | 0,01 bis 3,0 | 0,05 bis 2,5 | 0,1 bis 2,0 | 1,4 | - |
| optionale Additive | add 100 | add 100 | add 100 | add 100 | add 100 |

| | Formel 66 | Formel 67 | Formel 68 | Formel 69 | Formel 70 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| Silica Dimethyl Silylate | 0,01 bis 3,0 | 0,05 bis 2,5 | 0,1 bis 2,0 | 1,4 | - |
| optionale Additive | add 100 | add 100 | add 100 | add 100 | add 100 |

| | Formel 71 | Formel 72 | Formel 73 | Formel 74 | Formel 75 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| kationisches Tensid a3) | 0,01 bis 4,0 | 0,05 bis 2,0 | 0,1 bis 1,0 | 0,02 | 0,05 |
| hydrophobiertes Metalloxidpulver a4) | 0,01 bis 3,0 | 0,05 bis 2,5 | 0,1 bis 2,0 | 1,4 | 0,7 |
| optionale Additive | add 100 | add 100 | add 100 | add 100 | add 100 |

| | Formel 77 | Formel 77 | Formel 78 | Formel 79 | Formel 80 |
|---|---|---|---|---|---|
| Ethanol a1) | 20 bis 94 | 30 bis 89 | 40 bis 79 | 72 | 33 |
| Oryza Sativa (Rice) Starch | 5 bis 79 | 10 bis 69 | 20 bis 59 | 25 | 66 |
| kationisches Tensid a3) | 0,01 bis 4,0 | 0,05 bis 2,0 | 0,1 bis 1,0 | 0,02 | 0,05 |
| hydrophobiertes Metalloxidpulver a4) | 0,01 bis 3,0 | 0,05 bis 2,5 | 0,1 bis 2,0 | 1,4 | 0,7 |
| optionale Additive | add 100 | add 100 | add 100 | add 100 | add 100 |

| | Formel 81 | Formel 82 | Formel 83 | Formel 84 | Formel 85 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| (C₁₂ bis C₁₈)-Alkyltrimethylammoniumsalze | 0,01 bis 4,0 | 0,05 bis 2,0 | 0,1 bis 1,0 | 0,02 | 0,05 |
| silanisiertes pyrogenes Siliciumdioxid a4) | 0,01 bis 3,0 | 0,05 bis 2,5 | 0,1 bis 2,0 | 1,4 | 0,7 |
| optionale Additive | add 100 | add 100 | add 100 | add 100 | add 100 |

| | Formel 86 | Formel 87 | Formel 88 | Formel 89 | Formel 90 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| (C₁₂ bis C₁₈)-Alkyltrimethylammoniumsalze | 0,01 bis 4,0 | 0,05 bis 2,0 | 0,1 bis 1,0 | 0,02 | 0,05 |
| silanisiertes pyrogenes Siliciumdioxid a4) | 0,01 bis 3,0 | 0,05 bis 2,5 | 0,1 bis 2,0 | 1,4 | 0,7 |
| optionale Additive | add 100 | add 100 | add 100 | add 100 | add 100 |

| | Formel 91 | Formel 92 | Formel 93 | Formel 94 | Formel 95 |
|---|---|---|---|---|---|
| Ethanol a1) | 20 bis 94 | 30 bis 89 | 40 bis 79 | 73 | 33 |
| Stärkeverbindung a2) | 5 bis 79 | 10 bis 69 | 20 bis 59 | 26 | 66 |
| Cetyltrimethylammoniumsalze | 0,01 bis 4,0 | 0,05 bis 2,0 | 0,1 bis 1,0 | 0,02 | 0,05 |
| Silica Dimethyl Silylate | 0,01 bis 3,0 | 0,05 bis 2,5 | 0,1 bis 2,0 | 1,4 | 0,7 |
| optionale Additive | add 100 | add 100 | add 100 | add 100 | add 100 |

| | Formel 96 | Formel 97 | Formel 98 | Formel 99 | Formel 100 |
|---|---|---|---|---|---|
| Ethanol a1) | 20 bis 94 | 30 bis 89 | 40 bis 79 | 73 | 33 |
| Oryza Sativa (Rice) Starch | 5 bis 79 | 10 bis 69 | 20 bis 59 | 26 | 66 |
| Cetyltrimethylammoniumsalze | 0,01 bis 4,0 | 0,05 bis 2,0 | 0,1 bis 1,0 | 0,02 | 0,05 |
| Silica Dimethyl Silylate | 0,01 bis 3,0 | 0,05 bis 2,5 | 0,1 bis 2,0 | 1,4 | 0,7 |
| optionale Additive | add 100 | add 100 | add 100 | add 100 | add 100 |

Bevorzugte kosmetische Zubereitungen a) bestehen, bezogen auf ihr Gesamtgewicht, zu mindestens 80 Gew.-%, vorzugsweise mindestens 90 Gew.-% und insbesondere mindestens 97 Gew.-% aus den Bestanteilen a1) und a2). Werden in den erfindungsgemäßen kosmetischen Zubereitungen a) Wirk- oder Hilfsstoffe aus den Gruppe der kationischen Tenside a3) und/oder der hydrophob modifizierten Metalloxidpulver a4) eingesetzt, so ist es bevorzugt, dass die kosmetische Zubereitung a) bezogen auf ihr Gesamtgewicht, zu mindestens 80 Gew.-%, vorzugsweise mindestens 90 Gew.-% und insbesondere mindestens 97 Gew.-% aus den Bestanteilen a1), a2) sowie dem Bestandteil a3) und/oder a4) besteht.

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 40 bis 80 Gew.-% Ethanol;
a2) 20 bis 60 Gew.-% mindestens einer Stärkeverbindung als Prozessgut in einer Vorrichtung zur Entspannungsverdampfung der kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
   einem Vorratsbehälter für die kosmetische Zubereitung a), aus welchem die kosmetische Zubereitung a) in den Behälter b1) gelangen kann, wobei
   - der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
   - der Vorratsbehälter mindestens das Zehnfache Volumen, vorzugsweise mindestens das Fünfzigfache Volumen des Behälters b1) aufweist,
   - der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen.

Gegenstand der vorliegenden Anmeldung ist zudem die Verwendung eines erfindungsgemäßen Produkts zur Beaufschlagung keratinhaltiger Fasern, insbesondere menschlicher Haare, mit einer kosmetischen Zubereitung a) bzw. zur Reinigung keratinhaltiger Fasern, insbesondere menschlicher Haare.

Ein Verfahren zur Behandlung keratinhaltiger Fasern, insbesondere menschlicher Haare, bei welchem die keratinhaltigen Fasern mittels einer Vorrichtung zur Entspannungsverdampfung einer kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
   einem Vorratsbehälter für die kosmetische Zubereitung a), aus welchem die kosmetische Zubereitung a) in den Behälter b1) gelangen kann, wobei
   - der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
   - der Vorratsbehälter mindestens das Zehnfache Volumen, vorzugsweise mindestens das Fünfzigfache Volumen des Behälters b1) aufweist,
   - der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen; mit einer kosmetischen Zubereitung a) enthaltend bezogen auf ihr Gesamtgewicht a1) 40 bis 80 Gew.-% Ethanol; a2) 20 bis 60 Gew.-% mindestens einer Stärkeverbindung beaufschlagt werden.

Um eine ausreichende Sprühwirkung zu erzielen, wird die kosmetische Zubereitung a) vorzugsweise auf Temperaturen oberhalb des Siedepunkts des in der kosmetischen Zubereitung a) enthaltenen polaren Lösungsmittels oder Lösungsmittelgemisches erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

Bezüglich weiterer bevorzugter Ausführungsformen der erfindungsgemäßen Verwendungen und des erfindungsgemäßen Verfahrens gilt mutatis mutandis das zu den erfindungsgemäßen kosmetischen Zubereitungen a) und zu der Vorrichtung zur Entspannungsverdampfung b) Gesagte.

## Patentansprüche

1. Kosmetisches Produkt, umfassend
a) eine kosmetische Zubereitung, enthaltend bezogen auf ihr Gesamtgewicht
a1) 40 bis 80 Gew.-% Ethanol;
a2) 20 bis 60 Gew.-% mindestens einer Stärkeverbindung
b) eine Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
c) einen Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

2. Kosmetisches Produkt nach Anspruch 1, **dadurch gekennzeichnet, dass** die Stärkeverbindung a2) aus der Gruppe der - gegebenenfalls modifizierten - Polykondensationsprodukte von D-Glucose erhalten aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok.

3. Kosmetisches Produkt nach einem der vorherigen Ansprüche, **dadurch gekennzeichnet, dass** die kosmetische Zubereitung a) bezogen auf ihr Gesamtgewicht, 0,01 bis 4,0 Gew.-%, vorzugsweise 0,05 bis 2,0 Gew.-%, und insbesondere 0,1 bis 1,0 Gew.-% eines kationischen Tensids a3) enthält.

4. Kosmetisches Produkt nach einem der vorherigen Ansprüche, **dadurch gekennzeichnet, dass** die kosmetische Zubereitung a) bezogen auf ihr Gesamtgewicht, 0,01 bis 3,0 Gew.-%, vorzugsweise 0,05 bis 2,5 Gew.-% und insbesondere 0,1 bis 2,0 Gew.-% eines hydrophob modifizierten Metalloxidpulvers a4) enthält.

5. Verwendung einer kosmetischen Zubereitung a) enthaltend, bezogen auf ihr Gesamtgewicht,
a1) 40 bis 80 Gew.-% Ethanol;
a2) 20 bis 60 Gew.-% mindestens einer Stärkeverbindung
als Prozessgut in einer Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen.

6. Verwendung eines Produkts nach einem der Ansprüche 1 bis 4 zur Beaufschlagung keratinhaltiger Fasern, insbesondere menschlicher Haare, mit einer kosmetischen Zubereitung a).

7. Verwendung eines Produkts nach einem der Ansprüche 1 bis 4 zur Reinigung keratinhaltiger Fasern, insbesondere menschlicher Haare.

8. Verfahren zur Behandlung keratinhaltiger Fasern, insbesondere menschlicher Haare, bei welchem die keratinhaltigen Fasern mittels einer Vorrichtung zur Entspannungsverdampfung einer kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen;
mit einer kosmetischen Zubereitung a) enthaltend bezogen auf ihr Gesamtgewicht
a1) 40 bis 80 Gew.-% Ethanol;
a2) 20 bis 60 Gew.-% mindestens einer Stärkeverbindung
beaufschlagt werden.

9. Verfahren nach Anspruch 8, **dadurch gekennzeichnet, dass**
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter überführt wird;
- nachfolgend der Zugang zwischen Vorratsbehälter und Behälter durch ein Bauteil zur Durchflussregelung, mittels dessen der Durchfluss der kosmetischen Zubereitung a) aus dem Vorratsbehälter in den Behälter unterbrochen werden kann, unterbrochen wird;
- nachfolgend die in dem gegen die Umgebung abgeschlossenen Behälter befindliche kosmetische Zubereitung a) mittels einer Heizvorrichtung erhitzt wird, so dass der Druck im Inneren des Behälters auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter befindlichen kosmetischen Zubereitung aus dem Behälter in die Umgebung unter Minderung des zum Zeitpunkt der Behälteröffnung in dem Behälter herrschenden Drucks, entspannt wird.

## Claims

1. A cosmetic product, comprising
a) a cosmetic preparation containing, based on the total weight thereof,
a1) 40 to 80 wt.% of ethanol;
a2) 20 to 60 wt.% of at least one starch compound,
b) a device for flash evaporation of the cosmetic preparation a), comprising
b1) a container which defines a closed interior in which the cosmetic preparation can be received,
b2) a valve or a similarly functioning closure element for closing and opening the interior of the container that is at least partly filled with the cosmetic preparation a),
b3) a heating device for heating, under increased pressure, the cosmetic preparation a) which is located in the closed interior of the container, and releasing, under reduced pressure, the heated cosmetic preparation a) from the interior of the container into the surroundings,
b4) a nozzle which makes it possible to atomize the cosmetic preparation a) escaping from the container,
c) a reservoir for the cosmetic preparation a), from which the cosmetic preparation a) can enter the container b1), wherein
- the access between the reservoir and the container b1) has a component for flow control, by means of which component the flow of the cosmetic preparation a) from the reservoir into the container b1) can be interrupted,
- the reservoir has at least ten times the volume, preferably at least fifty times the volume of the container b1),
- the pressure inside the reservoir corresponds to the ambient pressure, and the cosmetic product does not comprise a propellant,
wherein the cosmetic product does not comprise a pump device which is suitable for releasing or spraying the cosmetic preparation a) without the action of the device for flash evaporation.

2. The cosmetic product according to claim 1, **characterized in that** the starch compound a2) from the group of optionally modified polycondensation products of D-glucose obtained from starch from potatoes, corn, rice, peas, acorns, chestnuts, barley, wheat, bananas, sago, millet, sorghum, oats, barley, rye, beans, sweet potato, arrowroot or cassava.

3. The cosmetic product according to one of the preceding claims, **characterized in that** the cosmetic preparation a) contains, based on the total weight thereof, 0.01 to 4.0 wt.%, preferably 0.05 to 2.0 wt.%, and in particular 0.1 to 1.0 wt.%, of a cationic surfactant a3).

4. The cosmetic product according to one of the preceding claims, **characterized in that** the cosmetic preparation a) contains, based on the total weight thereof, 0.01 to 3.0 wt.%, preferably 0.05 to 2.5 wt.%, and in particular 0.1 to 2.0 wt.%, of a hydrophobically modified metal oxide powder a4).

5. The use of a cosmetic preparation a) containing, based on the total weight thereof,
a1) 40 to 80 wt.% of ethanol;
a2) 20 to 60 wt.% of at least one starch compound,
as a process material in a device for flash evaporation of the cosmetic preparation a), comprising
b1) a container which defines a closed interior in which the cosmetic preparation can be received,
b2) a valve or a similarly functioning closure element for closing and opening the interior of the container that is at least partly filled with the cosmetic preparation a),
b3) a heating device for heating, under increased pressure, the cosmetic preparation a) which is located in the closed interior of the container, and releasing, under reduced pressure, the heated cosmetic preparation a) from the interior of the container into the surroundings,
b4) a nozzle which makes it possible to atomize the cosmetic preparation a) escaping from the container,
a reservoir for the cosmetic preparation a), from which the cosmetic preparation a) can enter the container b1), wherein
- the access between the reservoir and the container b1) has a component for flow control, by means of which component the flow of the cosmetic preparation a) from the reservoir into the container b1) can be interrupted,
- the reservoir has at least ten times the volume, preferably at least fifty times the volume of the container b1),
- the pressure inside the reservoir corresponds to the ambient pressure, and the cosmetic product does not comprise a propellant,
wherein no pump device is provided which is suitable for releasing or spraying the cosmetic preparation a) without the action of the device for flash evaporation.

6. The use of a product according to one of claims 1 to 4 for applying a cosmetic preparation a) to keratin fibers, in particular human hair.

7. The use of a product according to one of claims 1 to 4 for cleaning keratin fibers, in particular human hair.

8. A method for treating keratin fibers, in particular human hair, in which method, by means of a device for flash evaporation of a cosmetic preparation, said device comprising
b1) a container which defines a closed interior in which the cosmetic preparation can be received,
b2) a valve or a similarly functioning closure element for closing and opening the interior of the container that is at least partly filled with the cosmetic preparation a), b3) a heating device for heating, under increased pressure, the cosmetic preparation a) which is located in the closed interior of the container, and releasing, under reduced pressure, the heated cosmetic preparation a) from the interior of the container into the surroundings,
b4) a nozzle which makes it possible to atomize the cosmetic preparation a) escaping from the container,
a reservoir for the cosmetic preparation a), from which the cosmetic preparation a) can enter the container b1), wherein
- the access between the reservoir and the container b1) has a component for flow control, by means of which component the flow of the cosmetic preparation a) from the reservoir into the container b1) can be interrupted,
- the reservoir has at least ten times the volume, preferably at least fifty times the volume of the container b1),
- the pressure inside the reservoir corresponds to the ambient pressure, and the cosmetic product does not comprise a propellant,
wherein no pump device is provided which is suitable for releasing or spraying the cosmetic preparation a) without the action of the device for flash evaporation;
a cosmetic preparation a) containing, based on the total weight thereof,
a1) 40 to 80 wt.% of ethanol;
a2) 20 to 60 wt.% of at least one starch compound, is applied to the keratin fibers.

9. The method according to claim 8, **characterized in that**
- some of the cosmetic preparation a) located in a reservoir, in the interior of which a pressure prevails that corresponds to the ambient pressure, is transferred from said reservoir into a container;
- the access between the reservoir and the container is subsequently interrupted by means of a component for flow control, by means of which component the flow of the cosmetic preparation a) from the reservoir into the container can be interrupted;
- the cosmetic preparation a) located in the container, which is sealed against the surroundings, is subsequently heated by means of a heating device such that the pressure inside the container increases to values above the ambient pressure, preferably to values between 1.1 and 8 bar, in particular to values between 1.2 and 4 bar;
- the container, which is pressurized above the ambient pressure, is subsequently opened in a manner in which the discharge of at least some, preferably at least 50 wt.%, more preferably at least 80 wt.%, and in particular at least 90 wt.%, of the cosmetic preparation located in the container is released from the container into the surroundings by the pressure that prevails in the container at the time at which the container is opened being reduced.

## Revendications

1. Produit cosmétique, comprenant
a) une préparation cosmétique contenant, par rapport à son poids total,
a1) 40 à 80 % en poids d'éthanol ;
a2) 20 à 60 % en poids d'au moins un amylate
b) un dispositif de vaporisation éclair de la préparation cosmétique a) comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique a),
b3) un dispositif de chauffage permettant de chauffer la préparation cosmétique a) située dans l'espace intérieur fermé du récipient en augmentant la pression, ainsi que pour vaporiser la préparation cosmétique a) chauffée hors de l'espace intérieur du récipient dans l'environnement, en réduisant la pression,
b4) une buse permettant une atomisation de la préparation cosmétique a) s'échappant du récipient,
c) un récipient de stockage destiné à la préparation cosmétique a), à partir duquel la préparation cosmétique a) peut pénétrer dans le récipient b1),
- l'accès entre le récipient de stockage et le récipient b1) présentant un composant de régulation d'écoulement au moyen duquel l'écoulement de la préparation cosmétique a) du récipient de stockage vers le récipient b1) peut être interrompu,
- le récipient de stockage présentant au moins dix fois le volume, de préférence au moins cinquante fois le volume du récipient b1),
- la pression à l'intérieur du récipient de stockage correspondant à la pression ambiante et le produit cosmétique ne comprenant aucun agent gonflant,
dans lequel le produit cosmétique ne présente aucun dispositif de pompage adapté pour libérer ou pulvériser la préparation cosmétique a) sans l'action du dispositif de vaporisation éclair.

2. Produit cosmétique selon la revendication 1, **caractérisé en ce que** l'amylate a2) est choisi dans le groupe des produits de polycondensation - éventuellement modifiés - de D-glucose obtenus à partir d'amidon de pommes de terre, de maïs, de riz, de pois, de glands, de châtaignes, d'orge, de blé, de bananes, de sagou, de mil, de sorgho, d'avoine, de seigle, de haricots, de patates douces, de maranta ou de manioc ainsi que leurs mélanges.

3. Produit cosmétique selon l'une des revendications précédentes, **caractérisé en ce que** la préparation cosmétique a) contient, par rapport à son poids total, 0,01 à 4,0 % en poids, de préférence 0,05 à 2,0 % en poids et en particulier 0,1 à 1,0 % en poids d'un tensioactif cationique a3).

4. Produit cosmétique selon l'une des revendications précédentes, **caractérisé en ce que** la préparation cosmétique a) contient, par rapport à son poids total, 0,01 à 3,0 % en poids, de préférence 0,05 à 2,5 % en poids et en particulier 0,1 à 2,0 % en poids d'une poudre d'oxyde métallique hydrophobiquement modifiée a4).

5. Utilisation d'une préparation cosmétique a) contenant, par rapport à son poids total,
a1) 40 à 80 % en poids d'éthanol ;
a2) 20 à 60 % en poids d'au moins un amylate
en tant que matériau de processus dans un dispositif de vaporisation éclair de la préparation cosmétique a) comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique a),
b3) un dispositif de chauffage permettant de chauffer la préparation cosmétique a) située dans l'espace intérieur fermé du récipient en augmentant la pression, ainsi que pour vaporiser la préparation cosmétique a) chauffée hors de l'espace intérieur du récipient dans l'environnement, en réduisant la pression,
b4) une buse permettant une atomisation de la préparation cosmétique a) s'échappant du récipient,
un récipient de stockage destiné à la préparation cosmétique a), à partir duquel la préparation cosmétique a) peut pénétrer dans le récipient b1),
- l'accès entre le récipient de stockage et le récipient b1) présentant un composant de régulation d'écoulement au moyen duquel l'écoulement de la préparation cosmétique a) du récipient de stockage vers le récipient b1) peut être interrompu,
- le récipient de stockage présentant au moins dix fois le volume, de préférence au moins cinquante fois le volume du récipient b1),
- la pression à l'intérieur du récipient de stockage correspondant à la pression ambiante et le produit cosmétique ne comprenant aucun agent gonflant,
aucun dispositif de pompage adapté pour libérer ou pulvériser la préparation cosmétique a) sans l'action du dispositif de vaporisation éclair n'étant prévu.

6. Utilisation d'un produit selon l'une des revendications 1 à 4 permettant l'application d'une préparation cosmétique a) sur des fibres kératiniques, en particulier sur des cheveux humains.

7. Utilisation d'un produit selon l'une des revendications 1 à 4 permettant le nettoyage de fibres kératiniques, en particulier de cheveux humains.

8. Procédé de traitement de fibres kératiniques, en particulier de cheveux humains, dans lequel une préparation cosmétique est appliquée sur les fibres kératiniques au moyen d'un dispositif de vaporisation éclair, la préparation cosmétique comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique a),
b3) un dispositif de chauffage permettant de chauffer la préparation cosmétique a) située dans l'espace intérieur fermé du récipient en augmentant la pression, ainsi que pour vaporiser la préparation cosmétique a) chauffée hors de l'espace intérieur du récipient dans l'environnement, en réduisant la pression,
b4) une buse permettant une atomisation de la préparation cosmétique a) s'échappant du récipient,
un récipient de stockage destiné à la préparation cosmétique a), à partir duquel la préparation cosmétique a) peut pénétrer dans le récipient b1),
- l'accès entre le récipient de stockage et le récipient b1) présentant un composant de régulation d'écoulement au moyen duquel l'écoulement de la préparation cosmétique a) du récipient de stockage vers le récipient b1) peut être interrompu,
- le récipient de stockage présentant au moins dix fois le volume, de préférence au moins cinquante fois le volume du récipient b1),
- la pression à l'intérieur du récipient de stockage correspondant à la pression ambiante et le produit cosmétique ne comprenant aucun agent gonflant, aucun dispositif de pompage adapté pour libérer ou pulvériser la préparation cosmétique a) sans l'action du dispositif de vaporisation éclair n'étant prévu ;
comportant une préparation cosmétique a) contenant, par rapport à son poids total,
a1) 40 à 80 % en poids d'éthanol ;
a2) 20 à 60 % en poids d'au moins un amylate.

9. Procédé selon la revendication 8, **caractérisé en ce que**
- une quantité partielle de la préparation cosmétique a) présente dans un récipient de stockage, à l'intérieur duquel règne une pression égale à la pression ambiante, est transférée dans un récipient ;
- l'accès entre le récipient de stockage et le récipient est ensuite interrompu par un composant de régulation d'écoulement au moyen duquel l'écoulement de la préparation cosmétique a) du récipient de stockage vers le récipient peut être interrompu ;
- la préparation cosmétique a) présente dans le récipient fermé à l'environnement est ensuite chauffée au moyen d'un dispositif de chauffage de sorte que la pression à l'intérieur du récipient dépasse la pression ambiante pour atteindre de préférence des valeurs comprises entre 1,1 et 8 bar, en particulier entre 1,2 et 4 bar ;
- le récipient se trouvant à une pression supérieure à la pression ambiante est ensuite ouvert de manière à vaporiser au moins une quantité partielle, de préférence au moins 50 % en poids, de manière préférée au moins 80 % en poids et en particulier au moins 90 % en poids de la préparation cosmétique présente dans le récipient dans l'environnement, hors du récipient, en réduisant la pression régnant dans le récipient au moment de l'ouverture de celui-ci.
